# EUROPEAN PATENT SPECIFICATION

(11) **EP 4 504 166 B1**
(45) Date of publication and mention of the grant of the patent: **29.04.2026**
(21) Application number: 23713695.7
(22) Date of filing: 04.04.2023
(51) Int. Cl.: A61K 31/198, A61K 31/216, A61K 38/05, A61P 13/12, A61P 17/04, A61K 9/00

(54) **TOPICAL FORMULATION FOR USE IN THE TREATMENT OR PREVENTION OF ITCHY DRY SKIN**
TOPISCHE FORMULIERUNG ZUR VERWENDUNG BEI DER BEHANDLUNG ODER PRÄVENTION VON JUCKENDER TROCKENER HAUT
FORMULATION TOPIQUE DESTINÉE À ÊTRE UTILISÉE DANS LE TRAITEMENT OU LA PRÉVENTION DES DÉMANGEAISONS CUTANÉES SÈCHES

(30) Priority: 06.04.2022 EP 22166912
(43) Date of publication of application: 12.02.2025
(73) Proprietor: MC2 Therapeutics Limited, Guildford GU2 8XG (GB)
(72) Inventor: CRUTCHLEY, Nigel, Guildford GU2 8XG (GB); BONDEBJERG, Jon, Guildford GU2 8XG (GB)
(74) Representative: Budde Schou A/S
(86) International application number: PCT/EP2023/058880
(87) International publication number: WO 2023/194400

(56) References cited:
- WO-A1-2011/025107
- WO-A1-2016/086280
- WO-A1-2017/215723
- US-A1- 2015 250 846
- WESTBY ERIN P. ET AL: "A review of the management of uremic pruritus: current perspectives and future directions", ITCH, vol. 5, no. 3, 3 September 2020 (2020-09-03), pages e38 - e38, XP093129122, ISSN: 2380-5048, DOI: 10.1097/itx.0000000000000038
- PAPOIU ALEXANDRU D.P.: "Voxel-based morphometry and arterial spin labeling fMRI reveal neuropathic and neuroplastic features of brain processing of itch in end-stage renal disease - PMC", J NEUROPHYSIOL. 112(7), 18 June 2014 (2014-06-18), pages 1729 - 1738, XP093129142, Retrieved from the Internet <URL:https://www.ncbi.nlm.nih.gov/pmc/articles/PMC4157178/?report=printable> [retrieved on 20240208]
- ELKE WEISSHAAR ET AL: "Antipruritic effects of two different 5-HT3 receptor antagonists and an antihistamine in haemodialysis patients", EXPERIMENTAL DERMATOLOGY, BLACKWELL MUNSGAARD, COPENHAGEN; DK, vol. 13, no. 5, 4 May 2004 (2004-05-04), pages 298 - 304, XP071775470, ISSN: 0906-6705, DOI: 10.1111/J.0906-6705.2004.00184.X
- BRENNAN FRANK P. ET AL: "Chronic Pruritus: Histamine Is Not Always the Answer!", JOURNAL OF PAIN AND SYMPTOM MANAGEMENT., vol. 50, no. 4, 1 October 2015 (2015-10-01), US, pages 566 - 570, XP093129129, ISSN: 0885-3924, DOI: 10.1016/j.jpainsymman.2015.04.009

## Description

The present invention relates to a topical formulation as defined in the appended claims for use in the treatment or prevention of itchy dry skin caused by urea.

### BACKGROUND ART

Uremic pruritus (also known as chronic kidney disease associated pruritus) is one of the most distressing symptoms of renal failure. It affects up to 50% of patients in dialysis (I). The skin of the uremic patients is often pale, due to anaemia, dry and with scratch marks. The condition may include skin symptoms such as eczema, prurigo nodularis, nummular eczema and licinification. The subjective symptoms include intense itching and burning which may be localized or generalized. There have been many hypotheses for the etiology of the condition which have led to a number of systemic and topical treatments. In general, these treatments have little or no effect although some may help in subgroups of patients (I).

Metabolic and endocrine changes in uremic dialyzed patients are many and complex (2). The accumulation of urea in the body is one of the central metabolic changes in chronic renal disease. Urea is excreted both via the renal system and through the sweat (3). The concentration in sweat is 2-4 times higher than the serum concentration. This implies that high concentrations of urea can be present on the skin in uremic patients (4). Sometimes the concentration is so large that urea is visible as uremic frost.

The stratum corneum, the outer part of the skin, is composed of layers of keratinized epithelia, in the form of protein enriched corneocytes, embedded in a lipid intercellular matrix composed of ceramides, cholesterol and free fatty acids and mixed with natural moisturizing factors (NMFs) (5) (6). Fifty percent of the NMF is composed of amino acids resulting from the enzymatic break down of epidermal proteins particularly filaggrin (6), and its pivotal amino acid degradation products including histidine, trans-urocanic acid and pyrrolidone carboxylic acid (7). The remaining part of the NMF consists of components of sweat including electrolytes, lactic acid, urea, and to a minor extent additional amino acids. Besides having a moisturizing effect (water binding) the amino acids represent part of natural UV protection (trans urocanic acid) and function as a pH stabilizing factor.

Individuals with truncation mutations in the gene coding for filaggrin are strongly predisposed to a severe form of dry skin, ichthyosis vulgaris, and/or eczema. It has been shown that almost 50% of all severe cases of eczema may have at least one mutated filaggrin gene. The barrier defect seen in filaggrin null carriers also appears to lead to increased asthma susceptibility and exacerbations.

High levels of urea has for long time been used for its ability to denature proteins both in biochemical research and in industrial processes (8). The denaturing action of urea on globular proteins is due to the stabilization of the unfolded form of the protein molecule. Urea also has a direct concentration dependent effect on amino acids at room temperature (8). Small amounts of cyanate which will be present in aqueous urea can add to the -NH₂ groups of proteins and amino acids as well as to -SH groups present to yield carbamyl derivatives (9). It has been demonstrated that the logarithm of the rate constant for reaction between cyanate and amino acids are related linearly to the PKa value of the amino groups of the amino acids or polypeptides (15).

The increased concentration of urea in the stratum corneum in the uremic patient (urea coming from sweat) is therefore expected to have a profound impact on the on enzymic biological processes in the epidermis and stratum corneum. The excessively high degree of urea induced protein denaturation combined with the pH disruption negates the humectancy benefits obtained by more normal levels of urea. These effects have not been investigated as an etiological factor for uremic pruritus or seen as a new avenue for treatment of the condition. The denaturizing effects of urea, in the concentrations present in uremic patients, is expected to have a profound effect on the formation of the NMF. Denaturation of proteins (particularly filaggrin) must be expected to decrease the filaggrin derived amino acids that constitutes the majority of the NMF and pH regulatory mechanism in the stratum corneum. Furthermore, any available histidine will not be transformed to trans-urocanic acid because the needed enzyme (histidinase) may also be denatured by the high urea concentration in the skin of the uremic patients. Histidine itself will be changed by the direct action of urea on the molecule. These disturbances, which are expected to significantly affect the quantity and quality of the NMF, have not been investigated previously because the knowledge and dynamics of the formation and biological effects of the NMF is new.

Concerning the prior art US2016/0008297 discloses a topical formulation for use in the treatment or prevention of itchy skin associated with renal diseases. There is no disclosure of a nucleophilic attack for sequestering urea, and the amino acids used in the formulation are not included to act as nucleophiles.

US20150252035 mentions treatment of itch associated with uremic pruritus among several other skin disorders but the disclosed TRPV4 inhibitors cannot sequester urea by acting as nucleophiles.

US2009/0186853 discloses a histidine derivative and N-propanoyl derivatives of amino acids that may treat pruritus. However, there is no disclosure of treating itchy dry skin caused by sequestering urea with a nucleophile. On the contrary US2009/0186853 mentions that urea may be included in the formulations.

WO2017/215723 discloses topical administration of nucleophiles for the treatment of itchy dry skin caused by urea.

### DISCLOSURE OF THE INVENTION

The object of the present invention is to provide a formulation to be used in the treatment or prevention of itchy dry skin caused by urea with improved properties and which is gentle on the patient's skin.

Without being bound by any theory the present inventors have found that the undesired effects of excess urea accumulating on the skin of patients with uremic pruritus can be counteracted by the presence of a compound with a nucleophilic group. Consequently, the present invention relates to a topical formulation for use in the treatment or prevention of itchy dry skin caused by urea, wherein the topical formulation comprises a compound selected from the group consisting of Ala-Gln, Ala-Glu, and Gly-Arg. Compounds of the following formula 1 other than Ala-Gln, Ala-Glu, and Gly-Arg are not according to the claimed invention and are present for illustration purposes only. 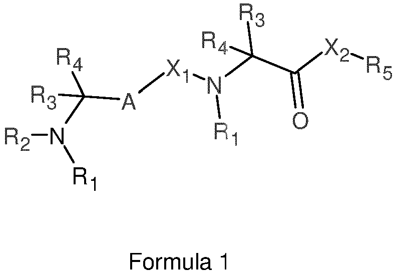
R₁ and R₂ are selected from H, C₁-C₆ alkyl, or R₁ or R₂ together with R₃ or R₄ forms a substituted or unsubstituted 3 to 6 membered ring
R₃ and R₄ are selected from H, C₁-C₆ alkyl, halogen, (C₁-C₆ alkyl)halogen, (C₁-C₆ alkyl)COOR₅, (C₁-C₆ alkyl)CONHR₅, (C₁-C₆ alkyl)NR₅R₅, (C₁-C₆ alkyl)OR₅, (C₁-C₆ alkyl)SR₅, substituted or unsubstituted (C₁-C₆ alkyl)aryl, substituted or unsubstituted (C₁-C₆ alkyl)heteroaryl, or R₃ or R₄ together with R₁ or R₂ forms a substituted or unsubstituted 3 to 6 membered ring, or R₃ and R₄ together forms a substituted or unsubstituted 3 to 6 membered ring
R₅ is selected from H or C₁-C₆ alkyl
A is (CHR₆)ₙ, wherein n = 0-2, and R₆ is selected from H, OH, OR₅, NR₅R₅, halogen, or C₁-C₆ alkyl
X₁ is selected from CHR₅ or CO
X₂ is selected from O or NH
and where A, R₁, R₂, R₃, R₄, R₅, R₆, X₁ and X₂ in each case are independently of one another and where at least one of the groups is a nucleophile at physiological conditions. More than one nucleophilic moiety, optionally more than one nucleophilic moiety of different types, may be present on the nucleophilic compound. In preferred embodiments the term 'substituted' means a group selected from C₁-C₆ alkyl, halogen, OR₅, OCF₃, NR₅R₅, cyano, (C₁-C₆ alkyl)halogen, (C₁-C₆ alkyl)COOR₅, (C₁-C₆ alkyl)CONHR₅, (C₁-C₆ alkyl)NR₅R₅, (C₁-C₆ alkyl)OR₅, (C₁-C₆ alkyl)SR₅

In preferred embodiments R₁ or R₂ together with R₃ or R₄ forms a 3 to 6 membered ring selected from the group of aziridinyl, azetidinyl, pyrrolidinyl, piperidinyl, tetrahydroimidazolyl, tetrahydrooxazolyl, tetrahydroisoxazolyl, tetrahydrothiazolyl, tetrahydropyrazolyl, piperazinyl or morpholinyl.

In preferred embodiments R₃ and R₄ together forms a 3 to 6 membered ring selected from the group of cyclopropyl, cyclobutyl, cyclopentyl, cyclohexyl, aziridinyl, azetidinyl, pyrrolidinyl, piperidinyl, piperazinyl, tetrahydroimidazolyl, tetrahydrooxazolyl, tetrahydroisoxazolyl, tetrahydrothiazolyl, tetrahydropyrazolyl, morpholinyl, oxiranyl, oxetanyl, tetrahydrofuranyl, tetrahydropyranyl, dioxanyl, thiiranyl, thietanyl, thiolanyl, or thianyl.

In embodiments of the topical formulation R₁ and R₂ are selected from H or methyl. In the topical formulation R₃ and R₄ are selected from H, C₁-C₆ alkyl, (C₁-C₆ alkyl)COOR₅, (C₁-C₆ alkyl)CONHR₅, (C₁-C₆alkyl)NR₅R₅, (C₁-C₆ alkyl)OR₅, substituted or unsubstituted (C₁-C₆ alkyl)aryl, substituted or unsubstituted (C₁-C₆ alkyl)heteroaryl.

The topical formulation for use also includes compounds wherein R₁ or R₂ together with R₃ or R₄ forms a substituted or unsubstituted 5 or 6 membered ring.

In embodiments of the topical formulation R₅ is H or methyl
The topical formulation for use includes embodiments, wherein n is 0 or 1, preferably n is 0.

In embodiments of the topical formulation for use R₆ is OH or OR₅, and preferably R₆ is OH.

The topical formulation also includes embodiments where X₁ is CO and X₂ is O.

The topical formulation for use also provides embodiments in which one of R₁ is H and R₂ is H, and the other R₁ together with R₃ or R₄ forms unsubstituted pyrrolidinyl; most preferably R₁ and R₂ is H.

The formula 1 may also include embodiments wherein one of R₃ or R₄ is H and the other is selected from H, C₁-C₆ alkyl, (C₁-C₆ alkyl)COOR₅, (C₁-C₆ alkyl)CONHR₅, (C₁-C₆ alkyl)NR₅R₅, (C₁-C₆ alkyl)OR₅, substituted or unsubstituted (C₁-C₆ alkyl)aryl, substituted or unsubstituted (C₁-C₆ alkyl)heteroaryl; preferably one of R₃ or R₄ is H and the other is C1-C6 alkyl or (C1-C6 alkyl)CONHR₅

The compounds described herein can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates. Wherein the compound is a naturally occurring dipeptide the preferred form is usually the L-configuration.

In a preferred embodiment, the compound and/or the pharmaceutically acceptable salt thereof has no more than three nucleophilic groups, more preferably no more than two nucleophilic groups and most preferentially has a single nucleophilic group.

In a preferred embodiment of the topical formulation for use according to the invention, the compound and/or the pharmaceutically acceptable salt thereof has molecular weight < 1000 Da. This, eases handling and manufacture of the formulation.

In a preferred embodiment of the topical formulation for use according to the invention, the compound and/or the pharmaceutically acceptable salt thereof also has an aqueous solubility greater than 1% (w/w). This, eases handling, manufacture of the formulation and allows suitable dosage levels to be achieved.

In a preferred embodiment of the topical formulation for use according to the invention, the compound and/or the pharmaceutically acceptable salt thereof also has stability in an aqueous medium of >80%, more preferably >90% after 1 month at 40°C. This enables formulation of an industrially marketable dosage form, as less than 20%, such as less than 10% of the compound has degraded after 1 month's storage in an aqueous medium at 40°C.

In a more preferred embodiment of the topical formulation for use according to the invention, the compound and/or the pharmaceutically acceptable salt thereof also has stability in an aqueous medium of >95%, after 3 month at 40°C. This enables formulation of a stable and industrially marketable dosage form, as less than 5%, of the compound has degraded after 3 month's storage in an aqueous medium at 40°C.

In a preferred embodiment at least one nucleophilic moiety is preferably in its unprotonated form.

The topical formulation for use in the treatment or prevention of itchy dry skin caused by urea and in embodiments the itchy dry skin caused by urea is selected from the group consisting of uremic pruritus, urostomy dermatitis, incontinence dermatitis, diaper dermatitis.

As stated, the present invention provides a topical formulation for use in the treatment of uremic pruritus. Specifically the invention provides a topical formulation for use for treating uremic pruritus of a subject, wherein the topical formulation comprising an active compound as defined in the appended claims, is applied to the skin with a concentration of the compound and/or a derivative thereof ranging between 0.001 mg/cm² and 5 mg/cm² skin surface, preferably between 0.003 mg/cm² and 1 mg/cm² skin surface, more preferably 0.005 mg/cm² and 0.5 mg/cm² skin surface. The compound may be the only active ingredient in the pharmaceutical composition. Alternatively, the formulation comprises more than one active compound of the formula 1.

The uremic pruritus is a well-defined indication, which can be clearly distinguished from inflammatory skin diseases (particularly a chronic inflammatory skin disease), such as atopic dermatitis, all types of psoriasis (including plaque flexural, guttate, pustular, nail, photosensitive, erythrodermic psoriasis and psoriatic arthritis), acne, ichthyosis, contact dermatitis, eczema, photodermatoses and dry skin disorders.

According to an embodiment the topical formulation can also be used in the treatment or prevention of pruritus in aging skin of elderly people.

In the topical formulation the active compound functions by inhibiting carbamylation in the patient's skin and preferably the compound results in a degree of BSA carbamylation of less than 80%, preferably less than 70%, more preferably less than 60%, even more preferably less than 50% as measured by the in vitro protein carbamylation assay provided herein.

The nucleophilic compound is believed to inhibit carbamylation by scavenging the cyanate/isocyanate via formation of a covalent bond between the nucleophile and cyanate/isocyanate. Preferably, said covalent bond is irreversible. The term irreversible as used herein refers to a reaction which does not revert, to any significant extent, back to the reactants under normal physiological conditions in the human body. Preferably the nucleophilic moiety is a primary amine.

In preferred topical formulations for use the compound is selected from the group consisting of N-β-aminoethyl-Gly, Lys-Pro, Val-Pro, Ile-Pro, Tyr-Pro, Ala-Gln, Ala-Glu, Tyr-Ala, Val-Tyr, Gly-Ser, Gly-His, and Aspartame.

In the topical formulation the compound and/or the pharmaceutically acceptable salt thereof is present in the topical formulation in an amount of between 0,1 %(w/w) to 20,0 %(w/w), preferably between 0,2 %(w/w) to 5,0 %(w/w), more preferably between 0,3 %(w/w) to 4,0 %(w/w) or between 0,5 %(w/w) to 20,0 %(w/w), preferably between 1 %(w/w) to 10 %(w/w), more preferably between 2 %(w/w) to 5,0 %(w/w).

### DETAILED DESCRIPTION OF THE INVENTION

The invention will now be described in further details with reference to some examples.

A number of compounds in accordance with formula 1 has been tested in an in vitro assay for their ability to inhibit urea-derived carbamylation of the model protein bovine serum albumin (BSA). The compounds of formula 1 according to the claimed invention are Ala-Gln, Ala-Glu, and Gly-Arg. References to other compounds of formula 1 relate to reference compounds and are present for illustration purposes only.

As evident from the below table a range of compounds in accordance with formula all have the ability to reduce or inhibit carbamylation of BSA, caused by urea. The tests were performed as described below.

200 µL of a 0.5 % BSA solution, 200 µL of test solution and 100 µL of a 5 µCi/mL 14C-Potassium Cyanate solution was mixed in an Eppendorf tube (corresponding to a final concentration of 75 µM BSA, 25 µM 14C-Potassium Cyanate, and 10 mM nucleophile). Samples run in triplicates (n=3). The Eppendorf tubes were incubated at 37 °C for 72 hours.

After incubation a volume of 100 µL of each sample was transferred to a new Eppendorf tube containing 100 µL of a 10 % trichloroacetic acid (TCA) and kept at 4 °C overnight (for precipitation of BSA). Subsequently, the precipitated BSA was separated from the buffer by centrifugation (10000 rpm, 30 seconds). The supernatant was removed and the BSA pellet 10 was resuspended in 200 µL of a 10 % solution of cold TCA (4 °C). The pellet was recovered by centrifugation (10000 rpm, 30 seconds) and supernatant was discarded. The BSA pellet was resuspended in 100 µL purified water (MilliQ) and transferred to a scintillation vial. The Eppendorf tube (previously containing the BSA pellet) was rinsed with an additional 100 µL purified water which al was transferred to the relevant scintillation vial. For scintillation 15 counting, 2 mL scintillation fluid (UltimaGold, PerkinElmer) was added to each scintillation vial and scintillation counting was done on a TRI-Carb 2910 Scintillation counter (PerkinElmer). Results for different compounds are shown in the table 1 and 2 below.

**Table 1. In vitro protein carbamylation assay results for different nucleophiles**

| **Compound** | **Degree of carbamylation (% relative to HBSS control)** | **SD** |
|---|---|---|
| HBSS control | 100 | 5.45 |
| N-β-aminoethyl-Gly | 29.7 | 0.34 |
| Lys-Pro | 28.6 | 0.75 |
| Val-Pro | 22.1 | 0.62 |
| Ile-Pro | 41.4 | 1.15 |
| Tyr-Pro | 32.8 | 0.88 |
| Ser-Pro | 26.4 | 0.57 |
| Pro-Ser | 41.9 | 1.15 |
| Ala-Gln | 34.8 | 0.54 |
| Ala-Glu | 20.4 | 0.20 |
| Tyr-Ala | 26.1 | 0.25 |
| Val-Tyr | 28.9 | 0.30 |
| Gly-Ser | 33.1 | 0.20 |
| Gly-His | 22.6 | 0.20 |
| Bestatin | 53.4 | 1.93 |
| Aspartame | 30.4 | 0.91 |
| | | |
| Arg | 52.4 | 1.45 |
| Arg*HCl | 66.1 | 3.89 |
| Histidine | 61.1 | 0.54 |
| | | |

**Table 2. In vitro protein carbamylation assay results for different nucleophiles**

| **Compound** | **Degree of carbamylation (% relative to HBSS control)** |
|---|---|
| HBSS Control | 100 |
| Ala-Gln | 34.8 |
| Ala-Glu | 20.4 |
| Ala(b)-His (L-Carnosine) | 72.63 |
| Gly-Ala | 35.27 |
| Gly-Gln | 32.99 |
| Gly-Gly | 26.6 |
| Gly-His | 22.6 |
| Gly-Ser | 33.1 |
| His-leu | 27.18 |
| Ile-Pro | 41.4 |
| Lys-Pro | 28.6 |
| Pro-Ser | 41.9 |
| Ser-Pro | 26.4 |
| Tyr-Ala | 26.1 |
| Tyr-Arg | 25.96 |
| Tyr-Pro | 32.8 |
| Val-Pro | 22.1 |
| Val-Tyr | 28.9 |
| | |
| Alanine | 93.54 |
| Arginine | 52.4 |
| Citrulline | 81.7 |
| Creatine | 103.92 |
| Glutamine | 74.39 |
| Histidine | 61.1 |
| Lysine | 71.63 |
| Ornithine | 57.98 |
| Taurine | 58.17 |
| | |

Thus, it is clear that compounds according to formula 1 with at least one nucleophilic group are capable of reducing carbamylation caused by urea. Further it is evident that the dipeptides and dipeptide derivatives usually give significantly greater efficacy than simple amino acids and thus are significantly more likely to be better urea derived isocyanate scavengers than the single amino acids.

As described above under the heading "Disclosure of the Invention" the inventors found that the site of action might be predominantly on the skin of the patient. Without being bound by theory, excess urea will be excreted predominantly from the sweat ducts and be potentially re-adsorbed. Treatment with a topical formulation is often associated with application to a damaged skin barrier which is highly likely for itchy dry skin caused by urea, because of the carbamylation and due to mechanical damage caused by the patient itching, such damaged skin barrier will be substantially more permeable than intact healthy skin. There exists a desire not to deliver the active compound transdermally. The patients in particular patients suffering from Uremic pruritus (Chronic Kidney Disease associated pruritus) already exhibit impaired kidney function and further stress is to be avoided if possible. However, to counteract the large concentration of urea and hence reactive isocyanate large doses of scavenger molecule are considered necessary.

Thus, in a preferred embodiment the compound has poor potential for transdermal permeation. Whereby the topical formulation for use in the treatment or prevention of itchy dry skin caused by urea, such as patients suffering from Uremic pruritus (Chronic Kidney Disease associated pruritus), is less likely to enter systemic circulation and thus less likely to cause further kidney stress.

In a more preferred embodiment the compound has poor potential for transdermal permeation and is actively converted to more benign degradants (i.e. amino acids), which are predominantly present endogenously Whereby the topical formulation for use in the treatment or prevention of itchy dry skin caused by urea, such as patients suffering from Uremic pruritus (Chronic Kidney Disease associated pruritus), is less likely to enter systemic circulation and thus less likely to cause further kidney stress. Furthermore, if the compound enters systemic circulation it will be less likely to cause further kidney stress due to its benign degradants and its exposure/concentration profile being less acute (Cₘₐₓ and Tₘₐₓ being suppressed). Notably the intravenous administration of the dipeptide Ala-Gln given as part of a clinical nutrition regimen in patients in hypercatabolic and/or hypermetabolic states is contraindicated for patients with severe renal insufficiency. (17)

It is well known in the art that compounds for a given molecular weight with more extreme Log P values are less likely to permeate the skin barrier well. Whether that be extremely hydrophilic (LogP <-2) or extremely hydrophobic (Log P >2) (16).) . All of the dipeptides have a molecular weight below 400 therefore a good approximation of their relative permeation potential is their LogP. According to the ACD/Labs calculated Log P values Ala-Gln is the one of the most hydrophilc in nature with a LogP of -4.4.

In one embodiment of the topical formulation for use according to the invention, the compound has a LogP value of less than -2, preferably less than -4.

In a preferred embodiment the compound is the dipeptide Ala-Gln.

To identify which of the dipeptides was readily subject to degradation by proteases and other cell processes in the skin, a number of compounds where tested in a skin homogenate study as described below and the results are listed in table 3.

### Skin homogenate study:

Dermatomed human skin from multiple skin donors was pooled, homogenized, and adjusted to a tissue concentration of 100 mg tissue per mL of homogenate. The homogenate was used immediately following preparation without any additional freeze/thaw cycles. Working solutions of each compound were prepared in Dulbecco's phosphate-buffered saline (DPBS) at 40 µg/mL. All solutions were preheated to 37°C. Equal volumes of skin homogenate and compound working solution were combined, mixed, held at 37°C, and sampled at 0, 15, 30, 60, 120, and 240 minutes. Samples were quenched with acidic acetonitrile, centrifuged, and prepared for LC-MS/MS analysis. Peak areas of each compound at each time-point were determined by LC-MS/MS.

The individual half-life of each dipeptide was determined.

**Table 3.**

| **Compound** | **Half life in skin homogenate (mins)** | **Log P (ACD Calculated)** | **Unsuitable*** |
|---|---|---|---|
| Ala-Ala | 23 | -1.16 | X |
| Ala-D-Ala | >480 | -1.16 | X |
| Ala-Gln | <5 | -4.4 | |
| Ala-Glu | 39 | -2.33 | |
| Ala-Gly | 60 | -1.5 | X |
| Ala-Gly (D) | >480 | -1.5 | X |
| Asp-Phe | 77 | 1.79 | X |
| Gly-Arg | 45 | -2.61 | |
| Gly-Asp | >480 | -1.49 | X |
| Gly-Gly | >480 | -0.89 | X |
| Gly-His | >480 | -2.04 | X |
| Gly-Pro | >480 | -1.34 | X |
| Gly-Sar | >480 | -1.42 | X |
| Gly-Val | 48 | 0.34 | X |
| Ile-Pro | 135 | 0.42 | X |
| Lys-Pro | 52 | -1.35 | X |
| Pro-Pro | 116 | -0.88 | X |
| Pro-Ser | 284 | -2.01 | X |
| Ser-Pro | 41 | -1.32 | X |
| Trp-Asn | 45 | -0.22 | X |
| Trp-Gly | 64 | 0.22 | X |
| Trp-Pro | 34 | 0.82 | X |
| Tyr-Ala | 50 | -0.1 | X |
| Tyr-Pro | 29 | 0.16 | X |
| Val-Pro | 39 | -0.11 | X |
| Val-Tyr | <7.5 | 0.83 | X |

| | | | |
|---|---|---|---|
| *Unsuitable - Defined as >50mins skin homogenate half life, or <95% assay after 3 months @40°C, poor solubility/too high a potential permeation (LogP >-2) | | | |

The nature of the experiment should be considered indicative of the relative stability of each of the compounds rather than an absolute correlation to what would happen *in vivo.* The process of homogenizing the skin is likely to greatly accelerate the degradation. However, it is clear that there is substantial variation in the relative rates for each dipeptide. (See Table 3)

Ala-Gln is one of the compounds with the shortest half-life measured (<5minutes) whilst some of the other dipeptides do not show any degradation during the experiment (480mins). This could be argued that this is advantageous to Ala-Gln as even though there is an endogenous amount in normal skin any compound that goes systemic after topical application is likely to be in the form of its constituent amino acids. The endogenous levels of singular amino acids are not likely to significantly altered even if all of the applied dose is split and enters the systemic system. Furthermore, the dipeptide being significantly more efficacious as a scavenger than its constituent amino acids cf. Table 1 and Table 2, one example being Ala-Gln having a degree of carbamylation (% relative to HBSS control) of 34.8 vs Alanine having a degree of carbamylation (% relative to HBSS control) of 93.54 and having a degree of carbamylation (% relative to HBSS control) of Glutamine 74.39.

Dipeptides can demonstrate significant variance in aqueous stability. An important aspect of formulating a stable topical product. Data in Table 4 below gives 1 month and 3 month stability data at 40°C. Compounds were dissolved at 3% wt/wt or 0.4% wt/wt (if 3% was not possible) in HBSS buffer and stored in glass vials at temperature. Samples were subsequently analyzed by HPLC-UV.

**Table 4. Aqueous stability data**

| **Compound** | **1 month aqueous stability at 40°C** | **3 month aqueous stability at 40°C** |
|---|---|---|
| Ala-Gln | 100.6 | 100.1 |
| Ala-Glu | 99.1 | 99.0 |
| Ala(b)-His (L-Carnosine) | 98.4 | 99.7 |
| Gly-Glu | 99.0 | 99.7 |
| Gly-His | 99.8 | 79.3 |
| Gly-Ser | 97.8 | 70.7 |
| Ile-Pro | 99.1 | 102.0 |
| Pro-Ser | 78.1 | 49.0 |
| Ser-Pro | 95.5 | 88.5 |
| Tyr-Ala | 100.4 | 94.0 |
| Val-Pro | 90.5 | 80.9 |
| Val-Tyr | 100.7 | 98.2 |

As can be appreciated from the data presented in table 1-4 above there is a large variability in performance for the different compounds making predictions difficult. Despite the apparent similarity between dipeptides surprisingly few of the possible combinations fulfil all the necessary performance criteria. The dipeptides Ala-Gln, Ala-Glu and Gly-Arg showed a combination of good scavenger potential, poor transdermal penetration potential, good aqueous stability, good solubility and rapid degradation *in vivo.* Furthermore, these dipeptides are based on natural amino acids which allows confidence in their toxicity profile as most natural amino acids as well as dipeptides thereof will be present at some endogenous level in healthy skin.

In normal skin many proteins involved in barrier formation are pH dependent and are only active in the UCA-created acidic environment of the upper epidermis/stratum corneum (10). In addition to maintaining this homeostasis, the lowered pH of the stratum corneum directly inhibits microbial infection and growth (11). It is known that defective filaggrin may result in reduced amino acid content including reduced levels of histidine in the stratum corneum, which in turn may lead to reduced levels of trans-UCA and an abnormally high pH. A higher than optimum pH in the stratum corneum is believed to reduce pH dependent lipid processing enzymes (for example β-glucocerebrosidase) and compromise barrier function and repair (12).

However, the present investigation is not concerned with solving the physiological cause of defective filaggrinin patients suffering from the skin disorders. The inventors have instead discovered that the use of a compound according to formula 1 and/or a derivative thereof as the active ingredient appears to compensate for defective filaggrin caused by the high concentration of urea in the skin of uremic pruritus patients. There currently exists very few means to prevent and/or treat uremic pruritus.

Moreover, when formula 1 results in dipeptides and/or a derivative thereof, such dipeptides exhibit low or no mammalian toxicity or reported side effects at conventional doses. Accordingly, the use of dipeptides including and/or a derivative thereof as the active ingredient provides advantages in use, including easy access to patients, improvement in patient compliance resulting in increasing usage of the active ingredients in wide patient populations and longer uninterrupted treatment regimens compared to alternative medicaments currently in use. Moreover, dipeptides and/or a derivative thereof are expected to be useful in treating the entire body surface of patients and to be effective against a wide range of skin disorders (especially inflammatory skin diseases). Preferably such skin diseases are caused by presence of urea or urine, and includes but are not limited to urostomi dermatitis, incontinence dermatitis, and diaper dermatitis, and more preferably uremic pruritus.

The novel observation made by the present inventors is that the high urea concentration is disturbing the normal function of proteins and amino acids. Moreover, the pH regulation is negatively affected by the high concentration of urea.

The compounds in accordance with formula 1 including dipeptides and/or a derivative thereof may be used as described herein in any suitable form, for example as discussed herein.

The compounds in accordance with formula 1 including dipeptides and/or a derivative thereof may be used as a sole therapy or in combination with a conventional therapy for the prevention and/or treatment of uremic pruritus. Suitable conventional therapies include, but are not limited to, treatment with steroids (for example steroids for topical administration) and/or with suitable lipids and/or with phototherapy.

The present invention further provides the topical formulation described above for use in the treatment or prevention of pruritus in aging skin of elderly people.

Pharmaceutical compositions for topical administration in accordance with the present invention may for example be in the form of solutions, creams, ointments, jellies, gels, sprays, foams, powders, liposomes, or aqueous or oily solutions or suspensions.

In the case of topical application to the scalp, the pharmaceutical composition may be formulated as a shampoo or conditioner. In the case of topical application to the skin, the pharmaceutical composition may be formulated as an additive to washing and bathing products (for example bath or shower gels and creams). Such pharmaceutical compositions for topical administration may include diluents or carriers that are also suitable for use in cosmetics.

Pharmaceutical compositions for topical administration by application to the skin may include moisturisers, and suntan lotions and creams.

A pharmaceutical composition for topical administration may be provided in any suitable dispenser.

In the case of pharmaceutical compositions for topical administration by application to the skin, the diluent or carrier should be selected so as to assist the transport of the active ingredient across the skin barrier and may need to be one capable of crossing the keratinous layer of the skin. Many methods are known for preparation of pharmaceutical compositions for topical application. For example, the active ingredient may be mixed with known carrier materials.

Alternatively, the skilled person will appreciate that topical administration may be achieved by means of localized injection, for example intra-dermal injection.

The pharmaceutical compositions of the invention may be obtained by conventional procedures using conventional pharmaceutical diluents or carriers, well known in the art.

### References

1 Fitzpatricks . Dermatology in general medicine. Eds.Freedberg IM et al. Six eds 2003,p 400.
2 Harrisons principels of internal medicine. Eds.Kasper et al 16th eds 2005. Chronic renal failure p 1653.
3 Fitzpatricks. Dermatology in general medicine.Eds.Freedberg IM et al six eds.2003,p103.
4 Al -Tamer YY,Hadi EA,al Badrani II.Ural.Res. 1997;25:337-40.
5 Fitzpatricks.Dermatology in general medicine.Eds.Freedberg IM et al. Six eds 2003,p 109.
6 Kezic S. Functions of filaggrin and its metabolites . In Flaggrin. Eds Thyssen JP and Maibach HI. 2014; p3-9.
7 Kezic S,O'Regan GM, YanN et al. Levels of filaggrin degradation products are influenced by both filaggrin genotype and atopic dermatitis severity. Allergy.2011; 66:934-940.
8 Nozaki Y and Tanford C. The solubility of amino acids and related compounds in aqueous urea solutions. J.Biol.Chem. 1963;238:4074-4081.
9 Stark G.R and Smyth D.G.The use of cyanate for the determination of NH2 - terminal residues in proteins.J.Biol.Chem.1963,238:214-226
10 Schmid-Wendtner MH and Korting HC, Skin Pharmacol. Physiol. 2006: 19:296-302
11 Rippke et al, Am. J. Clin. Dermatol. 2004; 5:217-23
12 Mauro et al, Arch. Dermatol. Res. 1998; 290: 215-22
13 Moss E et al. In situ metabolism of chinamyl alcohol in reconstructed human epidermis: New insigths into the activation of this fragrance sensitizer. Chem.Res.Toxicol. 2016; 29: 1172-8.
14 Bandier J et al. Quantification of epidermal filaggrin in human skin and its response to skin irritation. J.Invest Dermatol. 2015;136:1519-29.
15 G.R. Stark. Reactions of Cyanate with Functional Groups of Proteins. III. Reactions with Amino and Carboxyl Groups. Biochemistry 1965; 4: 1030-1036
16 Tsakovska I, Pajeva I, Al Sharif M, Alov P, Fioravanzo E, Kovarich S, Worth AP, Richarz AN, Yang C, Mostrag-Szlichtyng A, Cronin MTD. Quantitative structure-skin permeability relationships. Toxicology. 2017 Jul 15;387:27-42. doi: 10.1016/j.tox.2017.06.008. Epub 2017 Jun 21. PMID: 28645577
17 Dipeptevin Data sheet. https://www.fresenius-kabi.com/nz/documents/Dipeptiven_Datasheet.pdf

## Claims

1. Topical formulation for use in the treatment or prevention of itchy dry skin caused by urea, wherein the topical formulation comprises a compound selected from the group consisting of Ala-Gln, Ala-Glu, and Gly-Arg.

2. Topical formulation for use according to claim 1, wherein the compound is selected from the group consisting of Ala-Gln and Ala-Glu.

3. Topical formulation for use according to any one of the preceding claims, wherein the compound is Ala-Gln.

4. Topical formulation for use according to any one of the preceding claims, wherein the compound is Ala-Glu.

5. Topical formulation for use according to any one of the preceding claims, wherein the compound has a LogP value of less than -2; more preferably wherein the compound has a LogP value of less than -4.

6. Topical formulation for use according to any one of the proceeding claims, wherein the itchy dry skin caused by urea is selected from the group consisting of uremic pruritus, urostomy dermatitis, incontinence dermatitis, diaper dermatitis.

7. Topical formulation for use according to any one of the proceeding claims in the treatment or prevention of pruritus in aging skin of elderly people.

8. Topical formulation for use according to any one of claim 1 to claim 6 in the treatment or prevention of uremic pruritus.

9. Topical formulation for use according to any one of the preceding claims, wherein said compound results in a degree of BSA carbamylation of less than 80%, preferably less than 70%, more preferably less than 60%, even more preferably less than 50% as measured by the in vitro protein carbamylation assay provided herein.

10. Topical formulation for use according to any one of the preceding claims, wherein the compound and/or the pharmaceutically acceptable salt thereof is present in the topical formulation in an amount of between 0,1 %(w/w) to 20,0 %(w/w), preferably between 0,2 %(w/w) to 5,0 %(w/w), more preferably between 0,3 %(w/w) to 4,0 %(w/w).

11. Topical formulation for use according to any one of the preceding claims, wherein the compound and/or the pharmaceutically acceptable salt thereof is present in the topical formulation in an amount of between 0,5 %(w/w) to 20,0 %(w/w), preferably between 1 %(w/w) to 10 %(w/w), more preferably between 2 %(w/w) to 5,0 %(w/w).

12. Topical formulation for use according to any one of the preceding claims, wherein the compound has an aqueous stability greater than 95% after 3 months at 40°C.

## Patentansprüche

1. Topische Formulierung zur Verwendung bei der Behandlung oder Vorbeugung von juckender trockener Haut, die durch Harnstoff verursacht wird, wobei die topische Formulierung eine Verbindung umfasst, die aus der Gruppe ausgewählt ist, die aus Ala-Gln, Ala-Glu und Gly-Arg besteht.

2. Topische Formulierung zur Verwendung nach Anspruch 1, wobei die Verbindung aus der Gruppe ausgewählt ist, die aus Ala-Gln und Ala-Glu besteht.

3. Topische Formulierung zur Verwendung nach einem der vorhergehenden Ansprüche, wobei die Verbindung Ala-Gln ist.

4. Topische Formulierung zur Verwendung nach einem der vorhergehenden Ansprüche, wobei die Verbindung Ala-Glu ist.

5. Topische Formulierung zur Verwendung nach einem der vorhergehenden Ansprüche, wobei die Verbindung einen LogP-Wert von weniger als -2 aufweist; wobei die Verbindung vorzugsweise einen LogP-Wert von weniger als -4 aufweist.

6. Topische Formulierung zur Verwendung nach einem der vorangehenden Ansprüche, wobei die juckende trockene Haut, die durch Harnstoff verursacht wird, aus der Gruppe ausgewählt ist, die aus urämischem Pruritus, Urostomiedermatitis, Inkontinenzdermatitis, Windeldermatitis besteht.

7. Topische Formulierung zur Verwendung nach einem der vorangehenden Ansprüche bei der Behandlung oder Vorbeugung von Pruritus bei alternder Haut älterer Menschen.

8. Topische Formulierung zur Verwendung nach einem von Anspruch 1 bis Anspruch 6 bei der Behandlung oder Vorbeugung von urämischem Pruritus.

9. Topische Formulierung zur Verwendung nach einem der vorhergehenden Ansprüche, wobei die Verbindung zu einem Grad an BSA-Carbamylierung von weniger als 80 %, vorzugsweise weniger als 70 %, bevorzugter weniger als 60 %, noch bevorzugter weniger als 50 % führt, wie durch den hierin bereitgestellten In-vitro-Proteincarbamylierungs-Assay gemessen wird.

10. Topische Formulierung zur Verwendung nach einem der vorhergehenden Ansprüche, wobei die Verbindung und/oder das pharmazeutisch verträgliche Salz davon in der topischen Formulierung in einer Menge von zwischen 0,1 %(w/w) und 20,0 %(w/w), vorzugsweise zwischen 0,2 %(w/w) und 5,0 %(w/w), bevorzugter zwischen 0,3 %(w/w) und 4,0 %(w/w) vorhanden ist.

11. Topische Formulierung zur Verwendung nach einem der vorhergehenden Ansprüche, wobei die Verbindung und/oder das pharmazeutisch verträgliche Salz davon in der topischen Formulierung in einer Menge von zwischen 0,5 %(w/w) und 20,0 %(w/w), vorzugsweise zwischen 1 %(w/w) und 10 %(w/w), bevorzugter zwischen 2 %(w/w) und 5,0 %(w/w) vorhanden ist.

12. Topische Formulierung zur Verwendung nach einem der vorhergehenden Ansprüche, wobei die Verbindung eine wässrige Stabilität von mehr als 95 % nach 3 Monaten bei 40 °C aufweist.

## Revendications

1. Formulation topique pour utilisation dans le traitement ou la prévention des démangeaisons de la peau sèche prurigineuse provoquée par l'urée, dans laquelle la formulation topique comprend un composé choisi dans le groupe constitué par Ala-Gln, Ala-Glu et Gly-Arg.

2. Formulation topique pour utilisation selon la revendication 1, dans laquelle le composé est choisi dans le groupe constitué par Ala-Gln et Ala-Glu.

3. Formulation topique pour utilisation selon l'une quelconque des revendications précédentes, dans laquelle le composé est Ala-Gln.

4. Formulation topique pour utilisation selon l'une quelconque des revendications précédentes, dans laquelle le composé est Ala-Glu.

5. Formulation topique pour utilisation selon l'une quelconque des revendications précédentes, dans laquelle le composé présente une valeur de LogP inférieure à -2 ; plus préférablement dans laquelle le composé présente une valeur de LogP inférieure à -4.

6. Formulation topique pour utilisation selon l'une quelconque des revendications précédentes, dans laquelle la peau sèche prurigineuse provoquée par l'urée est choisie dans le groupe constitué de : prurit urémique, dermatite d'urostomie, dermatite d'incontinence, dermatite de couche.

7. Formulation topique pour utilisation selon l'une quelconque des revendications précédentes dans le traitement ou la prévention du prurit de la peau vieillissante des personnes âgées.

8. Formulation topique pour utilisation selon l'une quelconque des revendications 1 à 6 dans le traitement ou la prévention du prurit urémique.

9. Formulation topique pour utilisation selon l'une quelconque des revendications précédentes, dans laquelle ledit composé conduit à un degré de carbamylation de la BSA inférieur à 80 %, de préférence inférieur à 70 %, plus préférablement inférieur à 60 %, et encore plus préférablement inférieur à 50 %, tel que mesuré par le test de carbamylation des protéines in vitro fourni ici.

10. Formulation topique pour utilisation selon l'une quelconque des revendications précédentes, dans laquelle le composé et/ou le sel pharmaceutiquement acceptable de celui-ci est présent dans la formulation topique en une quantité comprise entre 0,1 % (p/p) et 20,0 % (p/p), de préférence entre 0,2 % (p/p) et 5,0 % (p/p), plus préférablement entre 0,3 % (p/p) et 4,0 % (p/p).

11. Formulation topique pour utilisation selon l'une quelconque des revendications précédentes, dans laquelle le composé et/ou le sel pharmaceutiquement acceptable de celui-ci est présent dans la formulation topique en une quantité comprise entre 0,5 % (p/p) et 20,0 % (p/p), de préférence entre 1 % (p/p) et 10 % (p/p), plus préférablement entre 2 % (p/p) et 5,0 % (p/p).

12. Formulation topique pour utilisation selon l'une quelconque des revendications précédentes, dans laquelle le composé présente une stabilité aqueuse supérieure à 95 % après 3 mois à 40 °C.
